# EUROPÄISCHE PATENTANMELDUNG

(11) **EP 4 696 683 A1**
(43) Veröffentlichungstag der Anmeldung: **18.02.2026**
(21) Anmeldenummer: 24194743.1
(22) Anmeldetag: 15.08.2024
(51) Int. Cl.: C07D 307/24

(54) **VERFAHREN ZUR HERSTELLUNG VON CIS-4-AMINOTETRAHYDROFURAN-2-CARBONSÄUREESTERN**

(71) Anmelder: Bayer Aktiengesellschaft, 51373 Leverkusen (DE)
(72) Erfinder: Die Erfindernennung liegt noch nicht vor
(74) Vertreter: BIP Patents

(57) **Zusammenfassung**

Die vorliegende Erfindung betrifft ein neues Verfahren zur Herstellung der Salze von cis-4-Aminotetrahydrofuran-2-carbonsäureester der allgemeinen Formel (I) in einem kontinuierlichen Verfahren ausgehen von einer Verbindung (II).

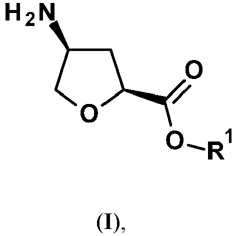

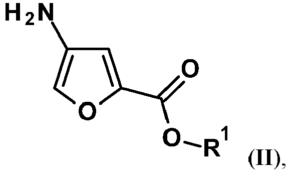

## Beschreibung

Die vorliegende Erfindung betrifft ein neues Verfahren zur Herstellung der Salze von cis-4-Aminotetrahydrofuran-2-carbonsäureestern der allgemeinen Formel **(I).**

Cis-4-Aminotetrahydrofuran-2-carbonsäuremethylester Hydrochlorid (CAS 1304126-28-0) der allgemeinen Formel **(I)** (mit R¹=Methyl) ist ein wichtiger Baustein zur Synthese von Pflanzenschutzmitteln (WO 2012/130798, WO 2021/170464).

Die erste publizierte Syntheseroute ist von Walker et al. (Synthesis 2011, 7, 1113-1119). Es wird 4-(*tert-*Butoxycarbonylamino)furan-2-carbonsäuremethylester (CAS 1170719-58-0, Wolter et al., Org. Lett. 2009, 11, 2804) als Vorstufe verwendet. 4-(*tert*-Butoxycarbonylamino)furan-2-carbonsäuremethylester ist allerdings für die großtechnische Synthese keine geeignete Ausgans- bzw. Zwischenverbindung: Es entstehen hohen Kosten, da die Reagenzien zur Herstellung sehr teuer sind, die Ausbeuten sind gering, die Sicherheitsrisiken sind hoch und es entstehen unerwünschte Phosphatabfällen, sodass die Herstellung im großtechnischen Maßstab nicht wirtschaftlich ist. Beispiele für diese nachteilige Herstellung sind eine Lithiierung mit *sec*-Butyllithium mit einer Aubeute von nur 34% und eine Curtius Umlagerung, für die DPPA (Diphenylphosphonazid) benötigt wird. Daher ist auch die Herstellung von *cis*-4-Aminotetrahydrofuran-2-carbonsäureestern im industriellen Maßstab über diese Vorstufe 4-(*tert-*Butoxycarbonylamino)furan-2-carbonsäuremethylester nicht umsetzbar.

Auch die Synthese über 4-Bromfuran-2-carbonsäuremethylester (CAS 58235-80-6, WO 2021/170464) ist aufgrund der Bromierung und der anschließenden ineffizienten Kupplung mit *tert*-Butylcarbamat (25% Ausbeute, WO 2021/170464) kostenintensiv und führt zu großen Mengen an umweltschädlichen Abfällen.

In WO 2023/161204 ist die Herstellung der Verbindungen der allgemeinen Formel **(I)** durch Hydrierung beschrieben. Ein Nachteil dieses Verfahrens ist die Bildung von Nebenkomponenten, wodurch die isolierte Ausbeute des gewünschten Produkts in hoher Reinheit nur bei 60-70% liegt.

Der vorliegenden Erfindung lag daher die Aufgabe zugrunde, den Prozess so zu verbessern, dass die Bildung von Nebenkomponenten reduziert und die isolierte Ausbeute des gewünschten Produkts gesteigert wird, während das Produkt in gleicher oder höherer Reinheit und zu geringeren Kosten erhalten wird.

Es wurde nun gefunden, dass die Bildung der Nebenkomponenten hauptsächlich durch intermolekulare Reaktion der Verbindungen der allgemeinen Formel **(II)** mit sich selbst stattfindet und durch lange Reaktionszeiten begünstigt wird. Lange Reaktionszeiten sind allerdings für einen hohen Umsatz bei kleiner Katalysatormenge im Batch-Verfahren aus ökonomischen Gründen notwendig.

Die zuvor beschriebene Aufgabe - einfache, kostengünstige und großtechnische Herstellung - wird gelöst durch ein Verfahren zur Herstellung von Verbindungen der allgemeinen Formel **(I)** in Form eines Salzes 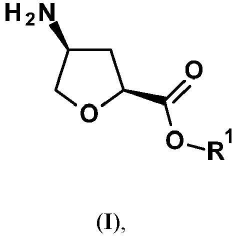 worin
- R¹: für (C₁-C₆)Alkyl oder (C₃-C₆)Cycloalkyl steht,
dadurch gekennzeichnet, dass Verbindungen der allgemeinen Formel **(II)** in Form eines Salzes 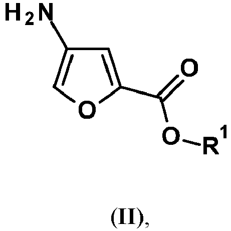 worin
- R¹: für (C₁-C₆)Alkyl oder (C₃-C₆)Cycloalkyl steht,
in einem Lösungsmittel gelöst werden und zusammen mit Wasserstoff kontinuierlich durch einen Reaktor gepumpt werden, der mit einem Rhodium-Katalysator befüllt ist.

In einer weiteren Ausführungsform wird dem Reaktionsgemisch eine Säure hinzugefügt.

Bevorzugte Restedefinitionen für die Verbindungen der allgemeinen Formeln **(I)** und **(II)** sind die folgenden:
- R¹: steht für (C₁-C₆)Alkyl.

Besonders bevorzugte Restedefinitionen für die Verbindungen der allgemeinen Formeln **(I)** und **(II)** sind die folgenden:
- R¹: steht für CH₃.

Die Reaktion zur Herstellung von Verbindungen der Formel **(I)** ist im Schema 1 dargestellt.

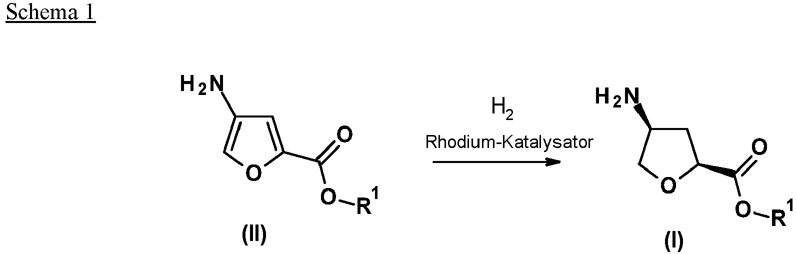

Die Verbindungen der allgemeinen Formel **(II)** in Form eines Salzes reagieren in Gegenwart von Wasserstoff, einem Rhodium Katalysator in einem Lösungsmittel zu Verbindungen der allgemeinen Formel **(I)** in Form eines Salzes.

Das erfindungsgemäße Verfahren kann in einem Rohrreaktor als Festbettreaktion mit fest angeordnetem Katalysator oder als Wirbelbettreaktion mit in auf- und abwirbelnder Bewegung befindlichem Katalysator oder in einem oder mehreren kontinuierlich gerührten Tankreaktoren durchgeführt werden. Bevorzugt ist die Durchführung im Festbettreaktor. Die Hydrierung wird in flüssiger Phase durchgeführt, beispielsweise in Riesel- oder Sumpffahrweise.

### Alternative

Ähnlich hohe Ausbeuten wie bei der kontinuierlichen Hydrierung erreicht man bei der Batch-Hydrierung, indem man die Katalysatormenge erhöht, sodass die Reaktionszeit kürzer wird. Damit diese Variante ökonomisch sinnvoll ist, muss der Katalysator mehrfach verwendet werden. Dafür muss die Reaktionslösung abgepumpt werden und frisches Edukt hinzugefügt werden. Diese Variante wurde bereits in WO 2023/161204 erwähnt, muss aber dahingehend verändert werden, dass eine höhere Katalysatormenge verwendet wird, damit die Reaktionszeit sinkt und damit auch die Bildung von Nebenkomponenten.

Der Druck bei der Reaktion beträgt 1 bis 100 bar, bevorzugt 5 bis 50 bar, besonders bevorzugt 20 bis 50 bar. Das molare Verhältnis von Wasserstoff zu Verbindungen der Formel **(II)** muss größer oder gleich zwei Äquivalente Wasserstoff pro Äquivalent der Verbindungen der Formel **(II)** sein; bevorzugt sind 2 bis 10 Äquivalente Wasserstoff; besonders bevorzugt sind 3 bis 5 Äquivalente Wasserstoff.

Die Temperatur bei der Reaktion beträgt 0°C bis 100 °C, bevorzugt 10°C bis 80°C, besonders bevorzugt 20 bis 40°C.

Als Katalysatoren können Rhodium-Katalysatoren oder gemischte Katalysatoren bestehend aus Rhodium und einem oder mehreren weiteren Metallen eingesetzt werden, wobei Rhodium und ggf. weitere Metalle auf einem Trägermaterial aufgebracht sind.

Als Trägermaterial können beispielsweise Aktivkohle, Aluminiumoxid, Siliciumdioxid, Siliciumcarbid, Calciumoxid, Titandioxid und/oder Zirkoniumdioxid verwendet werden; bevorzugt wird Aluminiumoxid verwendet.

Die Katalysatoren werden mit einer Edelmetall-Beladung von 0,1-10% Edelmetall auf dem Trägermaterial eingesetzt; bevorzugt werden 0,5-5% Edelmetall auf dem Trägermaterial aufgebracht. Die Verbindungen der Formel **(II)** werden in einer Katalysatorbelastung von 0,01-1 g (II)/g Katalysator/min zu Verbindungen der Formel **(I)** umgesetzt; bevorzugt werden 0,05-0,2 g (II)/g Katalysator/min zu Verbindungen der Formel **(I)** umgesetzt.

Eine Reaktivierung des Katalysators kann, beispielsweise durch geeignete Wäschen mit einem Lösungsmittel oder Lösungsmittelgemisch, vorteilhaft für dessen Wiederverwendung sein, beispielsweise durch Wäschen mit Methanol, Methanol/Dichlormethan oder saurem Methanol.

Die Verbindungen der Formel **(II)** werden als Salze ggf. mit einer Säure eingesetzt. Dem Reaktionsgemisch kann zusätzlich eine weitere Säure oder ein Säuregemisch hinzugefügt werden. Bevorzugt werden folgende Säuren zur Salzbildung mit Verbindungen der Formel **(II)** und/oder als Zusatz eingesetzt: HCl, H₂SO₄, MsOH, TfOH, TFA. Besonders bevorzugt ist HCl. Das molare Verhältnis der Säure zur Verbindung der Formel **(II)** liegt bei 0-100%, bevorzugt bei 0-10%.

Geeignete Lösungsmittel und Lösungsmittelgemische sind z.B.: R¹OH, R¹OH/Toluol, R'OH/Dichlormethan, bevorzugt sind R'OH und R¹OH/Dichlormethan, besonders bevorzugt sind Methanol und Methanol/Dichlormethan.

Fallen die Verbindungen der allgemeinen Formel (I) in Form ihrer Salze an, z.B. als Hydrochlorid, kann die salzfreie Form gewonnen werden, indem das Salz mit einer Base, z.B. Triethylamin, behandelt wird.

Verbindungen der allgemeinen Formel **(II)** werden gemäß WO 2022/018057 A1 hergestellt.

### Erläuterung des Verfahrens

### Beispiele

Die vorliegende Erfindung wird anhand der nachfolgenden Beispiele näher erläutert, ohne die Erfindung dabei auf diese einzuschränken.

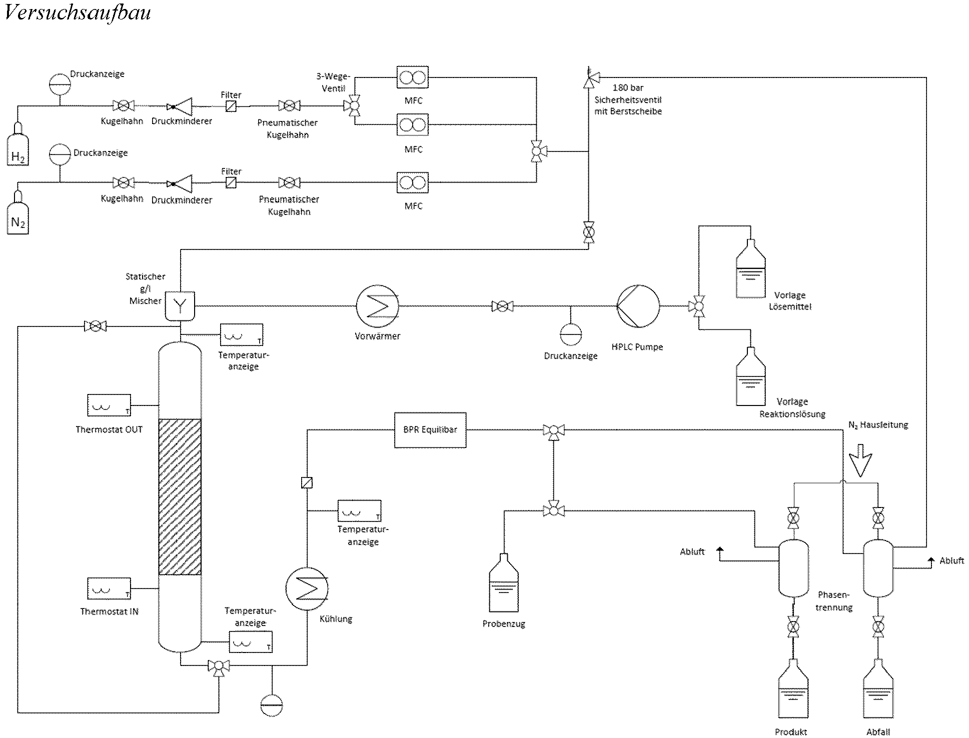

### Messverfahren

Die Produkte wurden mittels ¹H-NMR Spektroskopie und/oder GC/MS (Gas Chromatography Mass Spectrometry) charakterisiert.

Die NMR-Spektren wurden mit einem Bruker AV III 600 gemessen.

Die GC/MS Proben wurden mit einem Shimadzu GCMS-QP-2010-Ultra gemessen, das mit einem zusätzlichen FID (Flammen-Ionisations-Detektor) gekoppelt ist. Dazu wurden die Proben zunächst eingedampft und dann 1-10 mg trockene Probe zur Silylierung mit 250 µL N-Methyl-N-trimethylsilyltrifluoracetamid versetzt. Nach 1-5 Minuten Reaktionszeit wurden die Proben mit 1 ml Acetonitril verdünnt und gemessen.

Die quantitativen GC-Bestimmung wurden auf einer GC Agilent HB7890 B-Serie gemessen. Die Technik beruht auf GC mit FID Detektion, einer RTX-5 Amin Säule und mit interner Standard Auswertung (Interner Standard: Diethylphthalat). Die Proben, Referenzstandard, sowie interner Standard werden in Methanol gelöst.

### Herstellung von rac-cis-4-Aminotetrahydrofuran-2-carbonsäuremethylester Hydrochlorid

### Beispiel 1

Eine Lösung von 25 g 4-Aminofuran-2-carbonsäuremethylester Hydrochlorid (141 mmol) in 480 g Methanol und 120 g Dichlormethan wurde mit einem Fluss von 7 bis 15 ml/min gepumpt und in einem statischen Mischer mit Wasserstoff gemischt und dann durch einen auf 30°C beheizten, vertikal montierten Rohrreaktor (Durchmesser 9 mm, Länge 70 mm) abwärts gepumpt, der mit 4 g Rhodium-Katalysator (5% Rhodium auf Aluminiumoxid-Pulver) befüllt war. Der Gesamtdruck wurde über ein Druckhalteventil nach dem Rohrreaktor auf 30 bar eingestellt. Die erhaltene Reaktionsmischung wurde nach dem Druckhalteventil auf Umgebungsdruck entspannt, die Gasphase abgetrennt und die Reaktionslösung in Fraktionen gesammelt. Der Umsatz des Edukts zum gewünschten Zielprodukt wurde durch quantitative Gaschromatographie bestimmt.

**Tabelle 1**

| Fraktion | 0 | 1 | 2 | 3 | 4 | 5 | 6 |
|---|---|---|---|---|---|---|---|
| Zeitpunkt [min] | 0,0 | 5,0 | 15,0 | 22,5 | 30,0 | 40,0 | 50,0 |
| Flussrate Lösung [ml/min] | 0 | 15 | 15 | 15 | 15 | 7 | 7 |
| Verweilzeit Katalysatorbett [min] | 0,25 | 0,25 | 0,25 | 0,25 | 0,25 | 0,54 | 0,54 |
| Flussrate H2 [nL/h] | 0 | 15 | 15 | 15 | 15 | 7 | 7 |
| Edukt, quant. GC [%] | 3,81 | 0,02 | 0,03 | 0 | 0 | 0 | 0 |
| Produkt, quant. GC [%] | 0 | 3,57 | 3,61 | 3,65 | 3,6 | 3,57 | 3,48 |
| Umsatz Edukt [%] | 0 | 99,5 | 99,2 | 100,0 | 100,0 | 100,0 | 100,0 |
| in situ Ausbeute Produkt [%] | 0 | 91,6 | 92,7 | 93,7 | 92,4 | 91,6 | 89,3 |

### Beispiel 2

Eine Lösung von 41,7 g 4-Aminofuran-2-carbonsäuremethylester Hydrochlorid (219 mmol, Gehalt 93%) in 667 g Methanol und 333 g Dichlormethan wurde mit einem Fluss von 7,5 ml/min gepumpt und in einem statischen Mischer mit Wasserstoff (5 nL/h) gemischt und dann durch einen auf 30°C beheizten, vertikal montierten Rohrreaktor (Durchmesser 9 mm, Länge 700 mm) abwärts gepumpt, der mit 22,5 g Rhodium-Katalysator (3% Rhodium auf Aluminiumoxid-Kugeln, ca. 1 mm Durchmesser) und das restliche Reaktorvolumen mit 1,5 mm Glasperlen befüllt war. Der Gesamtdruck wurde über ein Druckhalteventil nach dem Rohrreaktor auf 30 bar eingestellt. Die Reaktionsmischung wurde nach dem Druckhalteventil auf Umgebungsdruck entspannt und die erhaltene Fraktion (902 g) unter reduziertem Druck auf 80 g aufkonzentriert. Die erhaltene Lösung wurde in einen Dreihalskolben mit KPG-Rührer überführt und mit 20 ml Methanol nachgespült. Zu der Lösung wurden 250 ml Toluol in 30 min bei Raumtemperatur zugetropft und die erhaltene Suspension dann über Nacht bei 0°C weiter gerührt. Die Suspension wurde über einen Nutsch-Filter abgesaugt und der erhaltene Feststoff unter reduziertem Druck getrocknet. Ausbeute der Fraktion: 33,5 g (81% der Theorie) der Zielverbindung.
Gehalt (quant. GC): 96,1% der Zielverbindung

¹H-NMR (DMSO-d6, 600 MHz): 1,98-2,05 (1H, m), 2,57-2,66 (1H, m), 3,69 (3H, s), 3,75-3,84 (2H, m), 3,91-3,98 (1H, m), 4,46-4,5 1fff (1H, t), 8,3-8,6 (3H, br) ppm.

GC/MS (m/z): 217 [M+TMS], 202, 187, 172, 158, 142, 128, 116, 100, 89, 73, 59, 54.

### Beispiel 3

Eine Lösung von 70 g 4-Aminofuran-2-carbonsäuremethylester Hydrochlorid (367 mmol, Gehalt 93%) in 1120 g Methanol und 560 g Dichlormethan wurde mit einem Fluss von 15 ml/min gepumpt und in einem statischen Mischer mit Wasserstoff (10 nL/h) gemischt und dann durch einen auf 30°C beheizten, vertikal montierten Rohrreaktor (Durchmesser 9 mm, Länge 700 mm) abwärts gepumpt, der mit 30,2 g Rhodium-Katalysator (3% Rhodium auf Aluminiumoxid-Kugeln, ca. 1 mm Durchmesser) und das restliche Reaktorvolumen mit 1,5 mm Glasperlen befüllt war. Der Gesamtdruck wurde über ein Druckhalteventil nach dem Rohrreaktor auf 30 bar eingestellt. Die Reaktionsmischung wurde nach dem Druckhalteventil auf Umgebungsdruck entspannt und die erhaltene Fraktion (1620 g) unter reduziertem Druck auf 210 g aufkonzentriert. Die erhaltene Lösung wurde in einen Dreihalskolben mit KPG-Rührer überführt. Zu der Lösung wurden 630 ml Toluol in 1 h bei 0°C zugetropft und die erhaltene Suspension dann 2 h bei 0°C weiter gerührt. Die Suspension wurde über einen Nutsch-Filter abgesaugt, 2 x mit 50 ml Toluol gewaschen und der erhaltene Feststoff unter reduziertem Druck getrocknet.
Ausbeute der Fraktion: 48,4 g (71% der Theorie) der Zielverbindung.
Gehalt (quant. GC): 97,7% der Zielverbindung.

### Beispiel 4

Eine Lösung von 29,99 g 4-Aminofuran-2-carbonsäuremethylester Hydrochlorid (157 mmol, Gehalt 93%) in 480 g Methanol und 240 g Dichlormethan wurde mit einem Fluss von 12 ml/min gepumpt und in einem statischen Mischer mit Wasserstoff (8 nL/h) gemischt und dann durch einen auf 30°C beheizten, vertikal montierten Rohrreaktor (Durchmesser 9 mm, Länge 700 mm) aufwärts gepumpt, der mit 30,2 g Rhodium-Katalysator (3% Rhodium auf Aluminiumoxid-Kugeln, ca. 1 mm Durchmesser) und das restliche Reaktorvolumen mit 1,5 mm Glasperlen befüllt war. Der Gesamtdruck wurde über ein Druckhalteventil nach dem Rohrreaktor auf 30 bar eingestellt. Die Reaktionsmischung wurde nach dem Druckhalteventil auf Umgebungsdruck entspannt und die erhaltene Fraktion (745 g) unter reduziertem Druck auf 90 g aufkonzentriert. Die erhaltene Lösung wurde in einen Dreihalskolben mit KPG-Rührer überführt. Zu der Lösung wurden 260 ml Toluol in 1 h bei 0°C zugetropft und die erhaltene Suspension dann 2 h bei 0°C weiter gerührt. Die Suspension wurde über einen Nutsch-Filter abgesaugt, 2 x mit 30 ml Toluol gewaschen und der erhaltene Feststoff unter reduziertem Druck getrocknet.
Ausbeute der Fraktion: 22,2 g (75,5% der Theorie) der Zielverbindung.
Gehalt (quant. GC): 97,0% der Zielverbindung.

## Patentansprüche

1. Verfahren zur Herstellung von Verbindungen der allgemeinen Formel (I) in Form eines Salzes 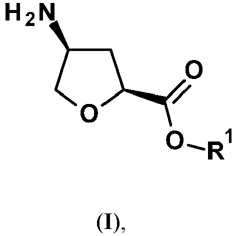 worin
R¹ für (C₁-C₆)Alkyl oder (C₃-C₆)Cycloalkyl steht,
**dadurch gekennzeichnet, dass** Verbindungen der allgemeinen Formel (II) in Form eines Salzes 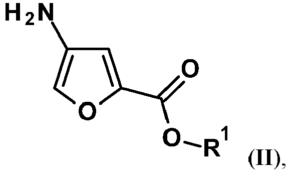 worin
R¹ für (C₁-C₆)Alkyl oder (C₃-C₆)Cycloalkyl steht,
in einem Lösungsmittel gelöst werden und zusammen mit Wasserstoff kontinuierlich durch einen Reaktor gepumpt werden, der mit einem Rhodium-Katalysator befüllt ist.

2. Verfahren nach Anspruch 1, **dadurch gekennzeichnet, dass** die Restedefinitionen für die Verbindungen der allgemeinen Formeln **(I)** und **(II)** die folgenden sind:
R¹ steht für (C₁-C₆)Alkyl.

3. Verfahren nach Anspruch 1, **dadurch gekennzeichnet, dass** die Restedefinitionen für die Verbindungen der allgemeinen Formeln **(I)** und **(II)** die folgenden sind:
R¹ steht für CH₃.

4. Verfahren nach einem der Ansprüche 1 bis 3, **dadurch gekennzeichnet, dass** dem Reaktionsgemisch eine Säure hinzugefügt wird.

5. Verfahren nach Anspruch 4, **dadurch gekennzeichnet, dass** zum Reaktionsgemisch eine weitere Säure oder Säuregemisch hinzugefügt wird.

6. Verfahren nach Anspruch 4 oder 5, **dadurch gekennzeichnet, dass** zum Reaktionsgemisch HCl als Säure hinzugefügt wird.

7. Verfahren nach einem der Ansprüche 1 bis 6, **dadurch gekennzeichnet, dass** R¹ Methyl entspricht und das Lösungsmittel Methanol ist.

8. Verfahren nach einem der Ansprüche 1 bis 6, **dadurch gekennzeichnet, dass** R¹ Methyl entspricht und das Lösungsmittel ein Lösungsmittelgemisch aus Dichlormethan und Methanol ist.

9. Verfahren nach einem der Ansprüche 1 bis 8, **dadurch gekennzeichnet, dass** 0.1 bis 10 Gew.% Katalysator (Trockengewicht) eingesetzt werden bezogen auf Verbindung der Formel (II).

10. Verfahren nach Anspruch 9, **dadurch gekennzeichnet, dass** als Katalysator Rh/C oder Rh/Alox verwendet wird.
